# EUROPEAN PATENT APPLICATION

(11) **EP 4 480 417 A1**
(43) Date of publication of application: **25.12.2024**
(21) Application number: 23180002.0
(22) Date of filing: 19.06.2023
(51) Int. Cl.: A61B 6/00

(54) **A MEDICAL SCAN PLANNING APPARATUS**

(71) Applicant: Koninklijke Philips N.V., 5656 AG Eindhoven (NL)
(72) Inventor: KROENKE-HILLE, Sven, Eindhoven (NL); SENEGAS, Julien Thomas, Eindhoven (NL); BERGTHOLDT, Martin, 5656AG Eindhoven (NL); BYSTROV, Daniel, Eindhoven (NL); MENSER, Bernd, Eindhoven (NL); DESHPANDE, Hrishikesh Narayanrao, Eindhoven (NL); PADALKO, Mikhail, Eindhoven (NL); YOUNG, Stewart Matthew, Eindhoven (NL)
(74) Representative: Philips Intellectual Property & Standards

(57) **Abstract**

The present invention relates to a medical scan planning apparatus, comprising: an input unit (10); a processing unit (20); and an output unit (30). The input unit (10) is configured to receive a first medical image (XI) of a patient. The input unit (10) is configured to receive a first 3D image (D1) of the patient, and wherein data of the first 3D image (D 1) was acquired at substantially the same time the first medical image (XI) was acquired. The input unit (10) is configured to receive a first geometrical relationship (G1) between an imaging system that acquired the first medical image (XI) and an imaging system that acquired the data of the first 3D image (D1). The input unit (10) is configured to receive a selection of a region of interest (RI) in the first medical image (XI). The processing unit is configured to convert the region of interest (RI) in the first medical image (XI) into a 3D region of interest (R2) in a coordinate system of the imaging system that acquired the data of the first 3D image (D 1), wherein the conversion comprises utilization of the first geometrical relationship (G1). The input unit (10) is configured to receive a second 3D image (D2) of the patient. The input unit (10) is configured to receive a second geometrical relationship (G2) between an imaging system to be used to acquire a second medical image (X2) of the patient and an imaging system that acquired the second 3D image (D2). The processing unit (20) is configured to determine at least one scan parameter for the imaging system to be used to acquire the second medical image (X2) of the patient, wherein the determination comprises utilization of the 3D region of interest (R2) in the coordinate system of the imaging system that acquired the data of the first 3D image (D1) and the second 3D image (D2) and the second geometrical relationship (G2). The output unit (30) is configured to output the at least one scan parameter for the imaging system to be used to acquire the second medical image (X2) of the patient.

## Description

### FIELD OF THE INVENTION

The present invention relates to a medical scan planning apparatus, a medical scan system, a medical scan planning method, a medical scan method, a computer program element, and a computer readable medium.

### BACKGROUND OF THE INVENTION

Appropriate scan-geometry planning is important for medical exams, such a CT or MR exams, to reduce dose (acquisition time) by imagining only the field-of-view which is relevant to answer the clinical question at hand and to save time. Typically, the scan-geometry is determined via localizer scans at a cost of additional dose and/or time resources.

There is a need to address these problems.

### SUMMARY OF THE INVENTION

It would be advantageous to have an improved technique to help plan a medical exam.

The object of the present invention is solved with the subject matter of the independent claims, wherein further embodiments are incorporated in the dependent claims.

In a first aspect, there is provided a medical scan planning apparatus, comprising:
- an input unit;
- a processing unit; and
- an output unit.

The input unit is configured to receive a first medical image of a patient. The input unit is configured to receive a first 3D image of the patient, and data of the first 3D image was acquired at substantially the same time the first medical image was acquired. The input unit is configured to receive a first geometrical relationship between an imaging system that acquired the first medical image and an imaging system that acquired the data of the first 3D image. The input unit is configured to receive a selection of a region of interest in the first medical image. The processing unit is configured to convert the region of interest in the first medical image into a 3D region of interest in a coordinate system of the imaging system that acquired the data of the first 3D image, and the conversion comprises utilization of the first geometrical relationship. The input unit is configured to receive a second 3D image of the patient. The input unit is configured to receive a second geometrical relationship between an imaging system to be used to acquire a second medical image of the patient and an imaging system that acquired the second 3D image. The processing unit is configured to determine at least one scan parameter for the imaging system to be used to acquire the second medical image of the patient. The determination comprises utilization of the 3D region of interest in the coordinate system of the imaging system that acquired the data of the first 3D image and the second 3D image and the second geometrical relationship. The output unit is configured to output the at least one scan parameter for the imaging system to be used to acquire the second medical image of the patient.

In an example, the imaging system that acquired the first medical image is: a stationary 2D X-ray unit, a mobile 2D X-ray unit, an ultrasound unit, a CT X-ray unit, or an MRI unit.

In an example, the imaging system to be used to acquire the second medical image of the patient is: a stationary 2D X-ray unit, a mobile 2D X-ray unit, an ultrasound unit, a CT X-ray unit, or an MRI unit.

In an example, the imaging system that acquired the data of the first 3D image was different to the imaging system that acquired the first medical image, and the data of the first 3D image was acquired by a 3D imaging system.

In an example, the 3D imaging system that acquired the data of the first 3D image was: a time-of-flight camera system, a stereo camera system, a LIDAR system, a radar system, an ultrasound system, or an RGB-D system.

In an example, the imaging system that acquired the data of the first 3D image was the same imaging system that acquired the first medical image, and the data of the first 3D image was derived by segmenting an outer surface of the patient in the first medical image.

In an example, the imaging system that acquired the second 3D image was: a time-of-flight camera system, a stereo camera system, a LIDAR system, a radar system, an ultrasound system, or an RGB-D system.

In an example, the at least one scan parameter comprises a 3D region of interest in the coordinate system of the imaging system to be used to acquire the second medical image of the patient.

In an example, the imaging system to be used to acquire the second medical image of the patient is an ultrasound system, and the at least one scan parameter comprises information to guide an ultrasound sensor to the required probe positioning and/or required probe angulation.

In an example, the at least one scan parameter comprises a kVp value for a CT scan or X-ray scan, and/or a tube current for a CT scan or X-ray scan.

In a second aspect, there is provided a medical scan system, comprising:
- an input unit;
- a processing unit;
- a 3D data acquisition system; and
- a medical image scanner.

The input unit is configured to receive a first medical image of a patient. The input unit is configured to receive a first 3D image of the patient, and data of the first 3D image was acquired at substantially the same time the first medical image was acquired. The input unit is configured to receive a first geometrical relationship between an imaging system that acquired the first medical image and an imaging system that acquired the data of the first 3D image. The input unit is configured to receive a selection of a region of interest in the first medical image. The processing unit is configured to convert the region of interest in the first medical image into a 3D region of interest in a coordinate system of the imaging system that acquired the data of the first 3D image, and the conversion comprises utilization of the first geometrical relationship. The medical image scanner is configured to acquire a second medical image of the patient. The 3D data acquisition system is configured to acquire a second 3D image of the patient. The processing unit is configured to access a second geometrical relationship between the medical image scanner to be used to acquire the second medical image of the patient and the 3D data acquisition system that acquired the second 3D image of the patient. The processing unit is configured to determine at least one scan parameter for the medical image scanner to be used to acquire the second medical image of the patient. The determination comprises utilization of the 3D region of interest in the coordinate system of the imaging system that acquired the data of the first 3D image and the second 3D image and the second geometrical relationship. The processing unit is configured to control the medical image scanner with respect to the at least one scan parameter for the medical image system to be used to acquire the second medical image of the patient.

In an example, the at least one scan parameter comprises a 3D region of interest in the coordinate system of the medical image scanner to be used to acquire the second medical image (X2) of the patient.

In a third aspect, there is provided a medical scan planning method, comprising:
- receiving by an input unit a first medical image of a patient;
- receiving by the input unit a first 3D image of the patient, and wherein data of the first 3D image was acquired at substantially the same time the first medical image was acquired;
- receiving by the input unit a first geometrical relationship between an imaging system that acquired the first medical image and an imaging system that acquired the data of the first 3D image;
- receiving by the input unit a selection of a region of interest in the first medical image;
- converting by a processing unit the region of interest in the first medical image into a 3D region of interest in a coordinate system of the imaging system that acquired the data of the first 3D image, wherein the converting comprises utilizing the first geometrical relationship;
- receiving by the input unit a second 3D image (D2) of the patient;
- receiving by the input unit a second geometrical relationship between an imaging system to be used to acquire a second medical image of the patient and an imaging system that acquired the second 3D image;
- determining by the processing unit at least one scan parameter for the imaging system to be used to acquire the second medical image of the patient, wherein the determining comprises utilizing the 3D region of interest in the coordinate system of the imaging system that acquired the data of the first 3D image and the second 3D image and the second geometrical relationship; and
- outputting by an output unit the at least one scan parameter for the imaging system to be used to acquire the second medical image of the patient.

In a fourth aspect, there is provided a medical scan method, comprising:
- receiving by an input unit a first medical image of a patient;
- receiving by the input unit a first 3D image of the patient, and wherein data of the first 3D image was acquired at substantially the same time the first medical image was acquired;
- receiving by the input unit a first geometrical relationship between an imaging system that acquired the first medical image and an imaging system that acquired the data of the first 3D image;
- receiving by the input unit a selection of a region of interest in the first medical image;
- converting by a processing unit the region of interest in the first medical image into a 3D region of interest in a coordinate system of the imaging system that acquired the data of the first 3D image, wherein the converting comprises utilizing the first geometrical relationship;
- acquiring by a 3D data acquisition system a second 3D image of the patient;
- accessing by the processing unit a second geometrical relationship between a medical image scanner to be used to acquire a second medical image of the patient and the 3D data acquisition system that acquired the second 3D image of the patient;
- determining by the processing unit at least one scan parameter for the medical image scanner to be used to acquire the second medical image of the patient, wherein the determining comprises utilizing the 3D region of interest in the coordinate system of the imaging system that acquired the data of the first 3D image and the second 3D image and the second geometrical relationship; and
- acquiring by the medical image scanner the second medical image of the patient comprising utilizing the at least one scan parameter for the medical image scanner to be used to acquire the second medical image of the patient.

In an aspect, there is provided a computer program element for controlling an apparatus according to the first aspect which when executed by a processor is configured to carry out the method of the fourth aspect.

In an aspect, there is provided a computer program element for controlling a system according to the second aspect which when executed by a processor is configured to carry out the method of the fourth aspect.

In an aspect, there is provided a computer program element for controlling a system according to the third aspect which when executed by a processor is configured to carry out the method of the fourth aspect.

Thus, according to aspects, there is provided computer program elements controlling one or more of the apparatuses/systems as previously described which, if the computer program element is executed by a processor, is adapted to perform the method as previously described.

According to another aspect, there is provided computer readable media having stored the computer elements as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawing:
Fig. 1 shows an example of a medical scan planning apparatus;
Fig. 2 shows an example of a medical scan system;
Fig. 3 shows an example of a medical scan planning method;
Fig. 4 shows an example of a medical scan method;
Fig. 5 shows a detailed workflow relating to a medical scan planning apparatus and/or to a medical scan system; and
Fig. 6 shows a pictorial representation of a detailed workflow relating to a medical scan planning apparatus and/or to a medical scan system.

### DETAILED DESCRIPTION OF EMBODIMENTS

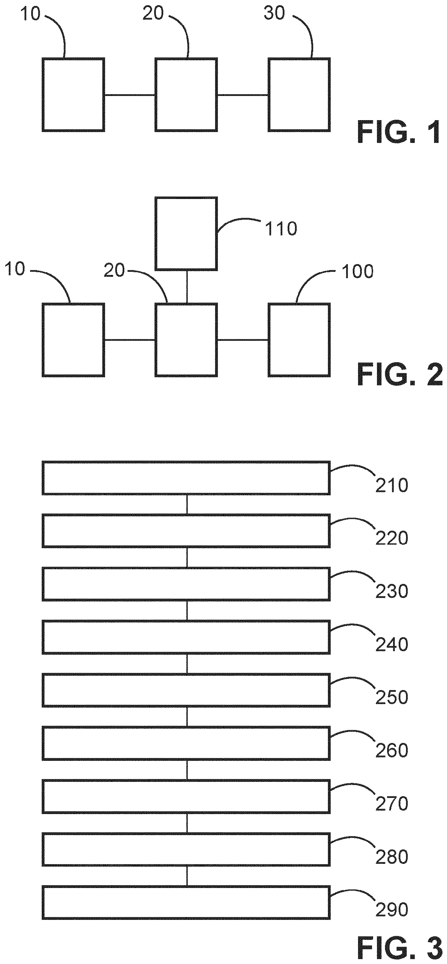

Fig. 1 shows an example of a medical scan planning apparatus, comprising:
- an input unit 10;
- a processing unit 20; and
- an output unit 30.

The input unit 10 is configured to receive a first medical image X1 of a patient. The input unit 10 is configured to receive a first 3D image D1 of the patient, and data of the first 3D image D1 was acquired at substantially the same time the first medical image X1 was acquired. The input unit 10 is configured to receive a first geometrical relationship G1 between an imaging system that acquired the first medical image X1 and an imaging system that acquired the data of the first 3D image D1. The input unit 10 is configured to receive a selection of a region of interest R1 in the first medical image X1. The processing unit is configured to convert the region of interest R1 in the first medical image X1 into a 3D region of interest R2 in a coordinate system of the imaging system that acquired the data of the first 3D image D1, and the conversion comprises utilization of the first geometrical relationship G1. The input unit 10 is configured to receive a second 3D image D2 of the patient. The input unit 10 is configured to receive a second geometrical relationship G2 between an imaging system to be used to acquire a second medical image X2 of the patient and an imaging system that acquired the second 3D image D2. The processing unit 20 is configured to determine at least one scan parameter for the imaging system to be used to acquire the second medical image X2 of the patient, and the determination comprises utilization of the 3D region of interest R2 in the coordinate system of the imaging system that acquired the data of the first 3D image D1 and the second 3D image D2 and the second geometrical relationship G2. The output unit 30 is configured to output the at least one scan parameter for the imaging system to be used to acquire the second medical image X2 of the patient.

Regarding the input unit, this could in effect be a part of the processing unit or a separate unit that provides data to the processing unit.

Regarding the output unit, this could in effect be a part of the processing unit or a separate unit that provides data to the processing unit.

It is to be noted that D1 can be acquired by any hardware, which can sense the patient's 3D surface (or segments thereof). This can involve a depth-camera using e.g. time-of-flight measurements or a stereo-camera configuration, a LIDAR or radar or ultra-sound systems. Also multiple of such sensors can be used if arranged in a known or measured spatial configuration. Alternatively, also RGB-D or only RGB camera(s) can be used (where the latter is equipped with an algorithm for estimating the depth maps from color images, see e.g. Fig. 7 in M.Oquab et al "DINOv2: Learning Robust Visual Features without Supervision" https://arxiv.org/pdf/2304.07193.pdf). Independently of how the depth measurement is implemented, one only needs to ensure that the first geometrical relationship G1 as well as the depth-measurement D1 is sufficiently accurate.

The first geometrical relationship G1 between the imaging system that acquired the first medical image X1 and the imaging system that acquired the data of the first 3D image D1 is important. This means that there should ideally be no patient motion in between the acquisition of X1 and D1, which can be easily realized by acquiring both images at the same time or so shortly after one-another in order that patient motion is negligible. However, if there is a longer waiting times between the acquisition of X1 and D1, potential patient motion (this can cover e.g. rigid motion, inhalation, changes in posture etc.) can be detected compensated (e.g. using an avatar model of the patient).

Similarly, the second geometrical relationship G2 between the imaging system to be used to acquire the second medical image X2 of the patient and the imaging system that acquired the second 3D image D2 is important, because patient motion can impact upon the estimated scan region R3 if the patient moves (in an unknown manner) between acquisition of the second 3D image D2 and the acquisition of the second medical image X2. Ideally, the patient is monitored by a depth-camera (or other 3D data acquisition system, similar) until the acquisition of X2 so that patient motion can be considered in the scan planning until the acquisition of X2. However, for CT / MRI scans, D2 can be acquired while the patient is lying on the table but not yet moved into the scanner, and then a known table movement of the table into the scanner can be taken into account.

In an example, the region of interest R1 in the first medical image X1 is selected by a medical professional.

According to an example, the imaging system that acquired the first medical image X1 is: a stationary 2D X-ray unit, a mobile 2D X-ray unit, an ultrasound unit, a CT X-ray unit, or an MRI unit.

According to an example, the imaging system to be used to acquire the second medical image X2 of the patient is: a stationary 2D X-ray unit, a mobile 2D X-ray unit, an ultrasound unit, a CT X-ray unit, or an MRI unit.

According to an example, the imaging system that acquired the data of the first 3D image D1 was different to the imaging system that acquired the first medical image X1, and the data of the first 3D image D1 was acquired by a 3D imaging system.

Thus for example, if the first medical image X1 was acquired by a CT or MRI unit, and the image was internal to the patient or only had a minor segment of the patient's skin, then a separate depth camera is used.

According to an example, the 3D imaging system that acquired the data of the first 3D image D1 was: a time-of-flight camera system, a stereo camera system, a LIDAR system, a radar system, an ultrasound system, or an RGB-D system.

According to an example, the imaging system that acquired the data of the first 3D image D1 was the same imaging system that acquired the first medical image X1, and the data of the first 3D image D1) was derived by segmenting an outer surface of the patient in the first medical image X1.

Thus for example, if the first medical image X1 was acquired by a CT or MRI unit, and the image covers a sufficiently large segment of the patient's surface (extreme case: full-body scan) then D1 can also be derived by segmenting the patient's skin surface in X1 (using e.g. intensity thresholding for CT or a CNN for MRI).

According to an example, the imaging system that acquired the second 3D image D2 was: a time-of-flight camera system, a stereo camera system, a LIDAR system, a radar system, an ultrasound system, or an RGB-D system.

According to an example, the at least one scan parameter comprises a 3D region of interest R3 in the coordinate system of the imaging system to be used to acquire the second medical image X2 of the patient.

Thus, the imaging system establishes a region in space to be scanned, and the patient is or will be located appropriately, and this region to be scanned corresponds to the region selected on a different image. In this way a scan such as a CT scan or MRI scan can be made more effectively and quicker, where the correct region of a patient to be scanned has been determined from an earlier acquired image. The CT scanner or MRI scanner is then used for less time, and for a CT scan X-ray dose for the patient can be minimised.

Ideally, the patient is monitored by a depth-camera (or other 3D data acquisition system. similar) until the acquisition of X2 so that patient motion can be considered in the scan planning until the acquisition of X2. However, for CT / MRI scans, D2 can be acquired while the patient is lying on the table but not yet moved into the scanner, and then a known table movement of the table into the scanner can be taken into account, and thus in this case, the movement of the table needs to be considered when estimating R3.

According to an example, the imaging system to be used to acquire the second medical image X2 of the patient is an ultrasound system, and the at least one scan parameter comprises information to guide an ultrasound sensor to the required probe positioning and/or required probe angulation.

Thus when R3 is defined for an ultrasound examination X2, the user needs to be guided to the desired US probe positioning, which can be done via projection on the patient's surface, augmented reality, audio guidance etc.

The at least one scan parameter can comprise information on an imaging plane, i.e. distance to the probe.

The information to guide an ultrasound sensor to the required probe positioning can be that the radiographer / radiologist needs to be guided to position the probe accordingly. Thus, this can be information that guides the radiographer / radiologist relating to the position on the patient's surface where the ultrasound probe needs to be positioned and/or the angulation of the ultrasound probe on the patient's surface. Thus, a feedback system can determine information on the position/angle of the probe and with respect to where the probe needs to be positioned and at what angle, guide the radiographer / radiologist accordingly.

According to an example, the at least one scan parameter comprises a kVp value for a CT scan or X-ray scan, and/or a tube current for a CT scan or X-ray scan.

Thus, the modalities for acquiring both X1 and X2 can be one of {X-ray (stationary and mobile systems), MRI, CT, Ultrasound} and the modality for X1 can be the same as or different to the modality for X2.

Fig. 2 shows an example of a medical scan system, comprising:
- an input unit 10;
- a processing unit 20;
- a 3D data acquisition system 100; and
- a medical image scanner 110.

The input unit 10 is configured to receive a first medical image X1 of a patient. The input unit 10 is configured to receive a first 3D image D1 of the patient, and data of the first 3D image D1 was acquired at substantially the same time the first medical image X1 was acquired. The input unit 10 is configured to receive a first geometrical relationship G1 between an imaging system that acquired the first medical image X1 and an imaging system that acquired the data of the first 3D image D1. The input unit 10 is configured to receive a selection of a region of interest R1 in the first medical image X 1. The processing unit is configured to convert the region of interest R1 in the first medical image X1 into a 3D region of interest R2 in a coordinate system of the imaging system that acquired the data of the first 3D image D1, and the conversion comprises utilization of the first geometrical relationship G1. The medical image scanner is configured to acquire a second medical image X2 of the patient. The 3D data acquisition system 100 is configured to acquire a second 3D image D2 of the patient. The processing unit 20 is configured to access a second geometrical relationship G2 between the medical image scanner to be used to acquire the second medical image X2 of the patient and the 3D data acquisition system that acquired the second 3D image D2 of the patient. The processing unit 20 is configured to determine at least one scan parameter for the medical image scanner to be used to acquire the second medical image X2 of the patient, and the determination comprises utilization of the 3D region of interest R2 in the coordinate system of the imaging system that acquired the data of the first 3D image D1 and the second 3D image D2 and the second geometrical relationship G2. The processing unit is configured to control the medical image scanner with respect to the at least one scan parameter for the medical image system to be used to acquire the second medical image (X2) of the patient.

In an example, the region of interest R1 in the first medical image X1 is selected by a medical professional.

In an example, the imaging system that acquired the first medical image X1 was: a stationary 2D X-ray unit, a mobile 2D X-ray unit, an ultrasound unit, a CT X-ray unit, or an MRI unit.

In an example, the medical image scanner to be used to acquire the second medical image (X2) of the patient is: a stationary 2D X-ray unit, a mobile 2D X-ray unit, an ultrasound unit, a CT X-ray unit, or an MRI unit.

In an example, the imaging system that acquired the data of the first 3D image D1 was different to the imaging system that acquired the first medical image X1, and the data of the first 3D image D1 was acquired by a 3D imaging system.

In an example, the imaging system that acquired the data of the first 3D image D1 was: a time-of-flight camera system, a stereo camera system, a LIDAR system, a radar system, an ultrasound system, or an RGB-D system.

In an example, the imaging system that acquired the data of the first 3D image D1 was the same imaging system that acquired the first medical image X1, and the data of the first 3D image D1 was derived by segmenting an outer surface of the patient in the first medical image X1.

In an example, the first medical image X1 was acquired by a CT or MRI unit,

In an example, the 3D data acquisition system 100 that acquired the second 3D image D2 is: a time-of-flight camera system, a stereo camera system, a LIDAR system, a radar system, an ultrasound system, or an RGB-D system.

According to an example, the at least one scan parameter comprises a 3D region of interest R3 in the coordinate system of the medical image scanner to be used to acquire the second medical image X2 of the patient.

Ideally, the patient is monitored by a depth-camera (or other 3D data acquisition system. similar) until the acquisition of X2 so that patient motion can be considered in the scan planning until the acquisition of X2. However, for CT / MRI scans, D2 can be acquired while the patient is lying on the table but not yet moved into the scanner, and then a known table movement of the table into the scanner can be taken into account, and thus in this case, the movement of the table needs to be considered when estimating R3.

In an example, the medical image scanner to be used to acquire the second medical image X2 of the patient is an ultrasound system, and the at least one scan parameter comprises information to guide an ultrasound sensor to the required probe positioning and/or required probe angulation.

The at least one scan parameter can comprise information on an imaging plane, i.e. distance to the probe.

In an example, the at least one scan parameter comprises a kVp value for a CT scan or X-ray scan, and/or a tube current for a CT scan or X-ray scan.

Fig. 3 shows a medical scan planning method 200, comprising:
- receiving 210 by an input unit 10 a first medical image X1 of a patient;
- receiving 220 by the input unit 10 a first 3D image D1 of the patient, and the data of the first 3D image D1 was acquired at substantially the same time the first medical image (X1) was acquired;
- receiving 230 by the input unit a first geometrical relationship G1 between an imaging system that acquired the first medical image X1 and an imaging system that acquired the data of the first 3D image D1;
- receiving 240 by the input unit 10 a selection of a region of interest R1 in the first medical image X1;
- converting 250 by a processing unit the region of interest R1 in the first medical image X1 into a 3D region of interest R2 in a coordinate system of the imaging system that acquired the data of the first 3D image D1, and the converting comprises utilizing the first geometrical relationship G1;
- receiving 260 by the input unit 10 a second 3D image D2 of the patient;
- receiving 270 by the input unit 10 a second geometrical relationship G2 between an imaging system to be used to acquire a second medical image X2 of the patient and an imaging system that acquired the second 3D image D2;
- determining 280 by the processing unit 20 at least one scan parameter for the imaging system to be used to acquire the second medical image X2 of the patient, and the determining comprises utilizing the 3D region of interest R2) in the coordinate system of the imaging system that acquired the data of the first 3D image D 1 and the second 3D image D2 and the second geometrical relationship G2; and
- outputting 290 by an output unit 30 the at least one scan parameter for the imaging system to be used to acquire the second medical image X2 of the patient.

In an example, the region of interest R1 in the first medical image X1 is selected by a medical professional.

In an example, the imaging system that acquired the first medical image X1 is: a stationary 2D X-ray unit, a mobile 2D X-ray unit, an ultrasound unit, a CT X-ray unit, or an MRI unit.

In an example, the imaging system to be used to acquire the second medical image X2 of the patient is: a stationary 2D X-ray unit, a mobile 2D X-ray unit, an ultrasound unit, a CT X-ray unit, or an MRI unit.

In an example, the imaging system that acquired the data of the first 3D image D1 was different to the imaging system that acquired the first medical image (X1), and the data of the first 3D image (D1) was acquired by a 3D imaging system.

In an example, the 3D imaging system that acquired the data of the first 3D image (D1 was: a time-of-flight camera system, a stereo camera system, a LIDAR system, a radar system, an ultrasound system, or an RGB-D system.

In an example, the imaging system that acquired the data of the first 3D image D1 was the same imaging system that acquired the first medical image X1, and the data of the first 3D image D 1 was derived by segmenting an outer surface of the patient in the first medical image X 1.

In an example, the imaging system that acquired the second 3D image D2 was: a time-of-flight camera system, a stereo camera system, a LIDAR system, a radar system, an ultrasound system, or an RGB-D system.

In an example, the at least one scan parameter comprises a 3D region of interest R3 in the coordinate system of the imaging system to be used to acquire the second medical image X2 of the patient.

In an example, the imaging system to be used to acquire the second medical image X2 of the patient is an ultrasound system, and the at least one scan parameter comprises information to guide an ultrasound sensor to the required probe positioning and/or required probe angulation .

In an example, the at least one scan parameter comprises a kVp value for a CT scan or X-ray scan, and/or a tube current for a CT scan or X-ray scan.

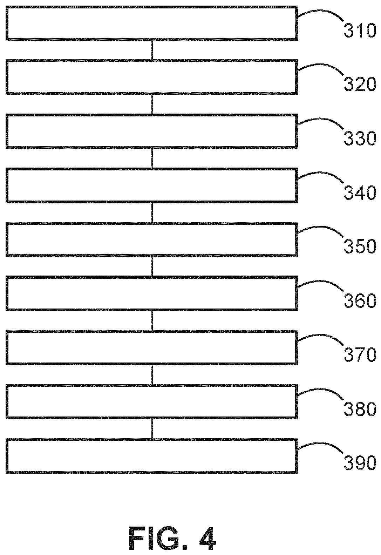

Fig. 4 shows a medical scan method 300, comprising:
- receiving 310 by an input unit 10 a first medical image X 1 of a patient;
- receiving 320 by the input unit 10 a first 3D image D1 of the patient, and data of the first 3D image D1 was acquired at substantially the same time the first medical image X1 was acquired;
- receiving 330 by the input unit 10 a first geometrical relationship G1 between an imaging system that acquired the first medical image X1 and an imaging system that acquired the data of the first 3D image D1;
- receiving 340 by the input unit 10 a selection of a region of interest R1 in the first medical image X1;
- converting 350 by a processing unit the region of interest R1 in the first medical image X1 into a 3D region of interest R2 in a coordinate system of the imaging system that acquired the data of the first 3D image D 1, and the converting comprises utilizing the first geometrical relationship G1;
- acquiring 360 by a 3D data acquisition system 100 a second 3D image D2 of the patient;
- accessing 370 by the processing unit 20 a second geometrical relationship G2 between a medical image scanner to be used to acquire a second medical image X2 of the patient and the 3D data acquisition system that acquired the second 3D image D2 of the patient;
- determining 380 by the processing unit 20 at least one scan parameter for the medical image scanner to be used to acquire the second medical image X2 of the patient, and the determining comprises utilizing the 3D region of interest R2 in the coordinate system of the imaging system that acquired the data of the first 3D image D1 and the second 3D image D2 and the second geometrical relationship G2; and
- acquiring 390 by the medical image scanner the second medical image X2 of the patient comprising utilizing the at least one scan parameter for the medical image scanner to be used to acquire the second medical image (X2) of the patient.

In an example, the region of interest R1 in the first medical image X1 is selected by a medical professional.

In an example, the imaging system that acquired the first medical image X1 was: a stationary 2D X-ray unit, a mobile 2D X-ray unit, an ultrasound unit, a CT X-ray unit, or an MRI unit.

In an example, the medical image scanner is: a stationary 2D X-ray unit, a mobile 2D X-ray unit, an ultrasound unit, a CT X-ray unit, or an MRI unit.

In an example, the imaging system that acquired the data of the first 3D image D1 was different to the imaging system that acquired the first medical image X1, and wherein the data of the first 3D image D1 was acquired by a 3D imaging system.

In an example, the imaging system that acquired the data of the first 3D image D1 was: a time-of-flight camera system, a stereo camera system, a LIDAR system, a radar system, an ultrasound system, or an RGB-D system.

In an example, the imaging system that acquired the data of the first 3D image D1 was the same imaging system that acquired the first medical image X1, and the data of the first 3D image D1 was derived by segmenting an outer surface of the patient in the first medical image X1.

In an example, the first medical image X1 was acquired by a CT or MRI unit,

In an example, the 3D data acquisition system 100 that acquired the second 3D image D2 is: a time-of-flight camera system, a stereo camera system, a LIDAR system, a radar system, an ultrasound system, or an RGB-D system.

In an example, the at least one scan parameter comprises a 3D region of interest R3 in the coordinate system of the medical image scanner to be used to acquire the second medical image X2 of the patient.

In an example, the medical image scanner is an ultrasound system, and the at least one scan parameter comprises information to guide an ultrasound sensor to the required probe positioning and/or required probe angulation.

In an example, the at least one scan parameter comprises a kVp value for a CT scan or X-ray scan, and/or a tube current for a CT scan or X-ray scan.

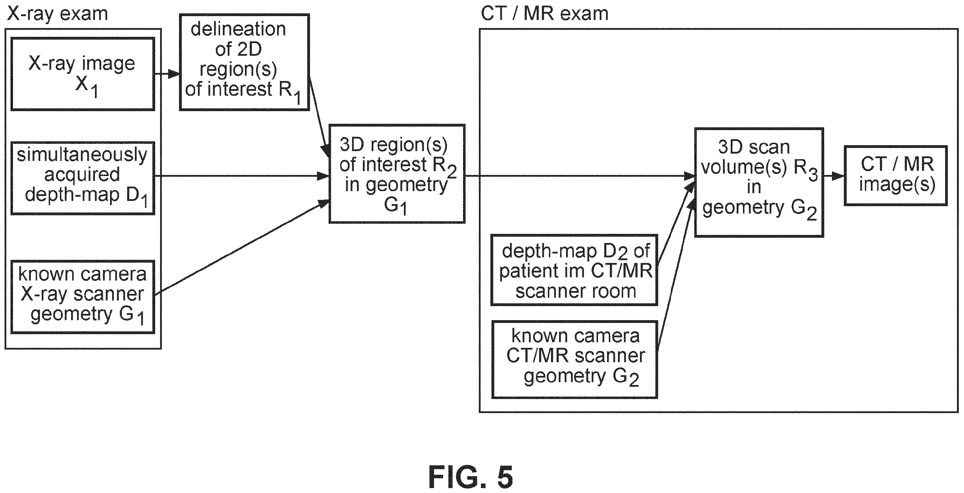

The medical scan planning apparatus, the medical scan system, the medical scan planning method, and the medical scan method are now described in specific detail, where reference is made to Figs. 5-6.

The following relates to utilization of a 2D X-ray image as a first image used to help plan the acquisition of a later CT image or a MRI image, but it is to be understood that the first image could be one acquired by a number of different modalities: a stationary 2D X-ray unit, a mobile 2D X-ray unit, an ultrasound unit, a CT X-ray unit, or an MIR unit. Also, the second later acquired image could be one acquired by a number of different modalities: a stationary 2D X-ray unit, a mobile 2D X-ray unit, an ultrasound unit, a CT X-ray unit, or an MRI unit. Below an MRI unit or MRI scan are also referred to as MR unit and MR scan.

As discussed above, appropriate scan-geometry planning is important for CT (MR) exams to reduce dose (acquisition time) by imagining only the field-of-view which is relevant to answer the clinical question at hand. Typically, the scan-geometry is determined via localizer scans at cost of additional dose and time resources.

It was realised by the inventors that (i) X-ray exams (or other image data) are often the entry point for further imaging exams and (ii) depth-cameras have become popular for assisting patient preparation. In such a scenario, the inventors realised that information obtained from the X-ray exam in combination with the recorded depth map can be utilized to plan a CT (MR) scan based on the actual indication in a patient-specific manner.

The new technique relates to a situation where an X-ray image in combination with a depth-map has already been acquired prior to the CT (MR) scan, which is typically ordered to verify, falsify, or differentiate indications for medical conditions provided by the X-ray image.

In doing this, the following benefits are provided:
A reduction in radiation dose for patients in CT scans, as a localizer scan not now being required.

A reduction in the technologist's time, as a localizer scan in CT / MR exams is not now required and other set-up efforts are not required.

The resultant CT/MR acquired data is more targeted leading to a reduction in scan-volumes and a reduction in the reading / reporting effort by radiologists.

Mitigation of communication barriers between the radiologists / physician, who has read the X-ray image and wants to order a CT / MR scan of a certain region, and the technologists operating the CT / MR scanner, which limits the specification of scan geometries, because the set up of the CT/MR scan is driven by the radiologist's targeted input/selection on an earlier X-ray image of the patient.

Overall, this reduces radiation dose for CT and time resources in CT / MR scans and enables to flexibly define patient- and indication-specific 3D scan geometries, which can also speed-up the reading and reporting by the radiologist.

Fig, 3 provides a schematic visualization of the main steps for medical scan planning provided by the new technique.

The medical scan planning apparatus, which can also be considered to be a system for the localizer-free CT / MR scan planning, consists of
An X-ray system with a depth-camera in known geometrical constellation, recording a depth-map at the time of X-ray acquisition;
A module to delineate one or more 2D regions of interests in the X-ray image;
   A module to derive 3D region(s) of interest from the recorded depth-map and the delineated 2D region(s) of interest;
A CT / MR scanner with a depth-camera in known geometrical constellation;
A module for mapping the 3D region(s) of interest into the CT / MR scanner geometry and the current patient's posture using its depth-camera signal;
The geometries of any required 3D scans are derived from the latter 3D region(s) of interests.

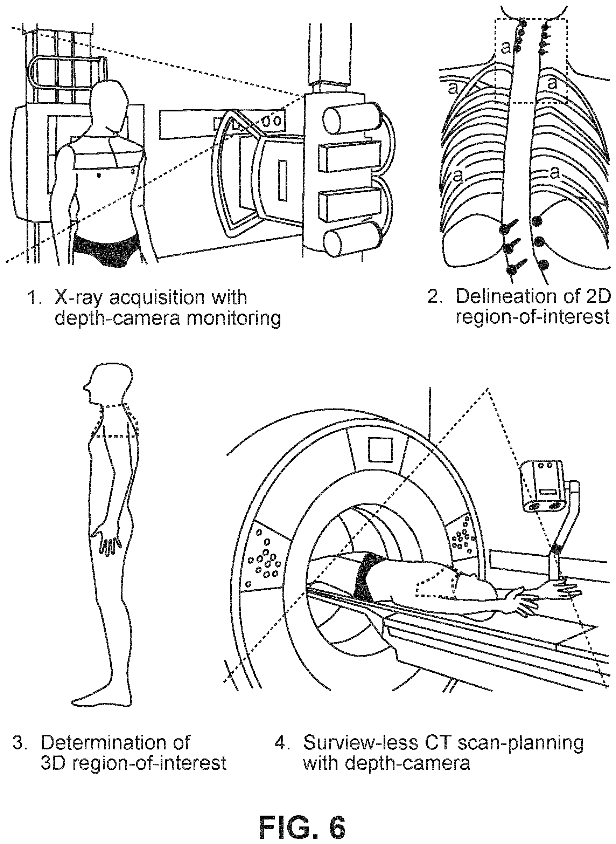

Referring to Fig. 5 and Fig. 6, the following steps apply:
At the time of the X-ray exam to acquire an X-ray image X1, a depth image D1 of the patient is recorded using a depth-camera with a known geometrical constellation G1 w.r.t. the X-ray detector and tube. G1 may be inferred from system parameters, sensors or from D1 by identifying e.g. markers attached to the detector. The depth image D 1 can be acquired simultaneously with the X-ray image X1 so that there is no movement of the patient between the two, but there can be a time delay between these and if it known how the patient has moved then the patient can even be intentionally moved between acquisition of D1 and X1.

If the patient is to be scheduled for a follow-up CT / MR scan, the 2D region of interest R1 is delineated by the radiographer or radiologist on the acquired X-ray image X1. Alternatively, R1 can be determined automatically by e.g. using a segmentation or bounding-box detection algorithm. This 2D region of interest R1 is mapped to a 3D volume of interest R2 using the depth-map D1 and the geometry G1.

When preparing the follow-up CT / MR exam, the 3D volume of interest R2 and the previously recorded depth-map D1 are loaded from a memory or hospital IT system. In the CT / MR exam room, the patient is observed by another depth camera with known or measured geometrical constellation G2 to the scanner. The thereby acquired depth map (frames) D2 is registered to D1 using e.g. a neural network for regression or the iterative-closes-point algorithm operating on the 3D point clouds. Alternatively, both D1 and D2 can be registered to an articulated (rigged) 3D human model (parameterized avatar, see http://www.makehumancommunity.org/) using (global or regional) rigid or deformable transformations. The thereby obtained transformation is used for mapping the 3D volume of interest R2 into the geometry G2, which finally results in the desired field-of-view R3 for the CT / MR scan.

Further embodiments are:
Organ and site-specific margins can be added to R2 or R3 to make sure that the anatomy of interest is not cropped in the field-of-view of the CT / MR scan.

Equipping the 3D avatar with anatomical information (e.g. likelihoods for organ positions) allows for restricting the 3D volume of interest R3 also along the direction of previous X-ray acquisition (i.e. the central-beam direction).

Gravitational sag leads to a different patient surface and organ constellation for CT / MR scans with the patient lying on the table as compared to X-ray exams with a wall-stand in front of which the patient stands. In particular for the abdomen, this can be taken into account when matching the depth maps D1 and D2. This can be accomplished by a statistical model inferred from pairs of full-body depth maps in up-right pose and MR scans of the same patient in combination with skin and organ segmentation. The 3D avatar can be equipped with this statistical model so that this shift of organ landmarks from a standing to a lying patient pose can be encoded in the estimated transformation from D1 to D2. Taking gravitational sag into account can be more important when the target anatomy of the CT / MR exam is soft-tissue as compared to exams targeting at bones.

Besides the field-of-view, also other scan parameters (such as the kVp or tube current for CT scans) can be estimated from D1, e.g. by estimating the relevant patient width in the volume of interest.

Position of the locator and/or tracker for CT contrast-enhanced studies also can be derived from the depth maps D 1 and D2 using the information about the anatomy of interest and the location of the ROI. E.g. if the liver is going to be scanned, locator should be set to descending aorta at the mid-lungs level; and if R1 corresponds to a multi-view scan, where for example multi-view relates to there being more than one image X1, the position of the tracker can also be estimated.

Specific embodiments of pathological anatomy which might also be automatically detected and delineated in the x-ray include bending or curvature of the spine, meaning that the margins of the 3D field-of-view (R2, R3) for a scan of the spine would need to be extended in the lateral directions accordingly. Another example can be pathological expansion of the lungs, meaning that the diaphragm is abnormally flattened or lowered in specific areas (e.g. costophrenic angles), such that the 3D FOV (R2, R3) should be extended caudally to ensure lower regions of the lungs are not clipped. In rare cases, the location of the heart is on the patient-right side, meaning once again that an adjustment of the 3D FOV would be required beyond the confines of regular variability of the typically observed locations.

If X-ray images are acquired for more than a single view on the anatomy (e.g. chest PA and LAT), a 2D region of interest can be manually or automatically delineated in each image (e.g. R1_{PA} and R1_{LAT}). The corresponding depth frames (e.g. D1_{PA} and D1_{LAT}) can then be employed to construct a 3D volume of interest R2 which can be more spatially confined as compared to the case where only one X-ray view is available (and information for the extent along the central-beam axis can only be incorporated via statistical models). This construction may involve joining these depth frames to a (at least partial view on the) 3D skin surface, e.g. using structure from motion techniques.

Thus, the new development relates to an apparatus/system for localizer-free CT and MR scan (and other imaging modality) planning which leverages the fact that X-ray exams (and other imaging modality image data) are often the entry point for further medical imaging. This new development in one embodiment is based on recording a depth-map of the patient at the instance of the X-ray acquisition with a depth-camera of known pose w.r.t. the X-ray system. Having delineated a desired 2D field-of-view in the acquired X-ray image, a corresponding 3D field-of-view is constructed using the recorded depth-map. During preparation of a follow-up CT or MR scan, another depth-camera with known pose w.r.t. the CT or MR scanner is used. The 3D scan volume is determined by registering the image of the second depth-camera to the depth-map corresponding to the X-ray acquisition and correspondingly transforming the desired 3D field-of-view. This summary focusses on the use of a 2D X-ray image to help in planning a subsequent CT or MR scan, but as discussed above the first image can be one of a number of different modalities as can the second image.

In another exemplary embodiment, a computer program or computer program element is provided that is characterized by being configured to execute the method steps of any of the methods according to one of the preceding embodiments, on an appropriate apparatus or system.

The computer program element might therefore be stored on a computer unit, which might also be part of an embodiment. This computing unit may be configured to perform or induce performing of the steps of the method described above. Moreover, it may be configured to operate the components of the above-described system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A medical scan planning apparatus, comprising:
- an input unit (10);
- a processing unit (20); and
- an output unit (30);
wherein the input unit (10) is configured to receive a first medical image (X1) of a patient;
wherein the input unit (10) is configured to receive a first 3D image (D1) of the patient, and wherein data of the first 3D image (D1) was acquired at substantially the same time the first medical image (X1) was acquired;
wherein the input unit (10) is configured to receive a first geometrical relationship (G1) between an imaging system that acquired the first medical image (X1) and an imaging system that acquired the data of the first 3D image (D1);
wherein the input unit (10) is configured to receive a selection of a region of interest (R1) in the first medical image (X1);
wherein the processing unit is configured to convert the region of interest (R1) in the first medical image (X1) into a 3D region of interest (R2) in a coordinate system of the imaging system that acquired the data of the first 3D image (D 1), wherein the conversion comprises utilization of the first geometrical relationship (G1);
wherein the input unit (10) is configured to receive a second 3D image (D2) of the patient;
wherein the input unit (10) is configured to receive a second geometrical relationship (G2) between an imaging system to be used to acquire a second medical image (X2) of the patient and an imaging system that acquired the second 3D image (D2);
wherein the processing unit (20) is configured to determine at least one scan parameter for the imaging system to be used to acquire the second medical image (X2) of the patient, wherein the determination comprises utilization of the 3D region of interest (R2) in the coordinate system of the imaging system that acquired the data of the first 3D image (D1) and the second 3D image (D2) and the second geometrical relationship (G2); and
wherein the output unit (30) is configured to output the at least one scan parameter for the imaging system to be used to acquire the second medical image (X2) of the patient.

2. Apparatus according to claim 1, wherein the imaging system that acquired the first medical image (X1) is: a stationary 2D X-ray unit, a mobile 2D X-ray unit, an ultrasound unit, a CT X-ray unit, or an MRI unit.

3. Apparatus according to any of claims 1-2, wherein the imaging system to be used to acquire the second medical image (X2) of the patient is: a stationary 2D X-ray unit, a mobile 2D X-ray unit, an ultrasound unit, a CT X-ray unit, or an MRI unit.

4. Apparatus according to any of claims 1-3, wherein the imaging system that acquired the data of the first 3D image (D1) was different to the imaging system that acquired the first medical image (X1), and wherein the data of the first 3D image (D1) was acquired by a 3D imaging system.

5. Apparatus according to claim 4, wherein the 3D imaging system that acquired the data of the first 3D image (D1) was: a time-of-flight camera system, a stereo camera system, a LIDAR system, a radar system, an ultrasound system, or an RGB-D system.

6. Apparatus according to any of claims 1-3, wherein the imaging system that acquired the data of the first 3D image (D1) was the same imaging system that acquired the first medical image (X1), and wherein the data of the first 3D image (D1) was derived by segmenting an outer surface of the patient in the first medical image (X1).

7. Apparatus according to any of claims 1-6, wherein the imaging system that acquired the second 3D image (D2) was: a time-of-flight camera system, a stereo camera system, a LIDAR system, a radar system, an ultrasound system, or an RGB-D system.

8. Apparatus according to any of claims 1-7, wherein the at least one scan parameter comprises a 3D region of interest (R3) in the coordinate system of the imaging system to be used to acquire the second medical image (X2) of the patient.

9. Apparatus according to any of claims 1-8, wherein the imaging system to be used to acquire the second medical image (X2) of the patient is an ultrasound system, and wherein the at least one scan parameter comprises information to guide an ultrasound sensor to the required probe positioning and/or required probe angulation.

10. Apparatus according to any of claims 1-8, wherein the at least one scan parameter comprises a kVp value for a CT scan or X-ray scan, and/or a tube current for a CT scan or X-ray scan.

11. A medical scan system, comprising:
- an input unit (10);
- a processing unit (20);
- a 3D data acquisition system (100); and
- a medical image scanner (110);
wherein the input unit (10) is configured to receive a first medical image (X1) of a patient;
wherein the input unit (10) is configured to receive a first 3D image (D1) of the patient, and wherein data of the first 3D image (D1) was acquired at substantially the same time the first medical image (X1) was acquired;
wherein the input unit (10) is configured to receive a first geometrical relationship (G1) between an imaging system that acquired the first medical image (X1) and an imaging system that acquired the data of the first 3D image (D1);
wherein the input unit (10) is configured to receive a selection of a region of interest (R1) in the first medical image (X1);
wherein the processing unit is configured to convert the region of interest (R1) in the first medical image (X1) into a 3D region of interest (R2) in a coordinate system of the imaging system that acquired the data of the first 3D image (D1), wherein the conversion comprises utilization of the first geometrical relationship (G1);
wherein the medical image scanner is configured to acquire a second medical image (X2) of the patient;
wherein the 3D data acquisition system (100) is configured to acquire a second 3D image (D2) of the patient;
wherein the processing unit (20) is configured to access a second geometrical relationship (G2) between the medical image scanner to be used to acquire the second medical image (X2) of the patient and the 3D data acquisition system that acquired the second 3D image (D2) of the patient;
wherein the processing unit (20) is configured to determine at least one scan parameter for the medical image scanner to be used to acquire the second medical image (X2) of the patient, wherein the determination comprises utilization of the 3D region of interest (R2) in the coordinate system of the imaging system that acquired the data of the first 3D image (D1) and the second 3D image (D2) and the second geometrical relationship (G2); and
wherein the processing unit is configured to control the medical image scanner with respect to the at least one scan parameter for the medical image system to be used to acquire the second medical image (X2) of the patient.

12. System according to claim 11, wherein the at least one scan parameter comprises a 3D region of interest (R3) in the coordinate system of the medical image scanner to be used to acquire the second medical image (X2) of the patient.

13. A medical scan planning method (200), comprising:
- receiving (210) by an input unit (10) a first medical image (X1) of a patient;
- receiving (220) by the input unit (10) a first 3D image (D1) of the patient, and wherein data of the first 3D image (D1) was acquired at substantially the same time the first medical image (X1) was acquired;
- receiving (230) by the input unit a first geometrical relationship (G1) between an imaging system that acquired the first medical image (X1) and an imaging system that acquired the data of the first 3D image (D1);
- receiving (240) by the input unit (10) a selection of a region of interest (R1) in the first medical image (X1);
- converting (250) by a processing unit the region of interest (R1) in the first medical image (X1) into a 3D region of interest (R2) in a coordinate system of the imaging system that acquired the data of the first 3D image (D1), wherein the converting comprises utilizing the first geometrical relationship (G1);
- receiving (260) by the input unit (10) a second 3D image (D2) of the patient;
- receiving (270) by the input unit (10) a second geometrical relationship (G2) between an imaging system to be used to acquire a second medical image (X2) of the patient and an imaging system that acquired the second 3D image (D2);
- determining (280) by the processing unit (20) at least one scan parameter for the imaging system to be used to acquire the second medical image (X2) of the patient, wherein the determining comprises utilizing the 3D region of interest (R2) in the coordinate system of the imaging system that acquired the data of the first 3D image (D1) and the second 3D image (D2) and the second geometrical relationship (G2); and
- outputting (290) by an output unit (30) the at least one scan parameter for the imaging system to be used to acquire the second medical image (X2) of the patient.

14. A medical scan method (300), comprising:
- receiving (310) by an input unit (10) a first medical image (X1) of a patient;
- receiving (320) by the input unit (10) a first 3D image (D1) of the patient, and wherein data of the first 3D image (D1) was acquired at substantially the same time the first medical image (X1) was acquired;
- receiving (330) by the input unit (10) a first geometrical relationship (G1) between an imaging system that acquired the first medical image (X1) and an imaging system that acquired the data of the first 3D image (D1);
- receiving (340) by the input unit (10) a selection of a region of interest (R1) in the first medical image (X1);
- converting (350) by a processing unit the region of interest (R1) in the first medical image (X1) into a 3D region of interest (R2) in a coordinate system of the imaging system that acquired the data of the first 3D image (D1), wherein the converting comprises utilizing the first geometrical relationship (G1);
- acquiring (360) by a 3D data acquisition system (100) a second 3D image (D2) of the patient;
- accessing (370) by the processing unit (20) a second geometrical relationship (G2) between a medical image scanner to be used to acquire a second medical image (X2) of the patient and the 3D data acquisition system that acquired the second 3D image (D2) of the patient;
- determining (380) by the processing unit (20) at least one scan parameter for the medical image scanner to be used to acquire the second medical image (X2) of the patient, wherein the determining comprises utilizing the 3D region of interest (R2) in the coordinate system of the imaging system that acquired the data of the first 3D image (D1) and the second 3D image (D2) and the second geometrical relationship (G2); and
- acquiring (390) by the medical image scanner the second medical image (X2) of the patient comprising utilizing the at least one scan parameter for the medical image scanner to be used to acquire the second medical image (X2) of the patient.

15. A computer program element for controlling an apparatus according to any of claims 1-10 which when executed by a processor is configured to carry out the method of claim 13, or for controlling a system according to any of claims 11-12 which when executed by a processor is configured to carry out the method of claim 14.
